# EUROPEAN PATENT APPLICATION

(11) **EP 4 442 227 A1**
(43) Date of publication of application: **09.10.2024**
(21) Application number: 24168464.6
(22) Date of filing: 04.04.2024
(51) Int. Cl.: A61F 5/02

(54) **ORTHOPAEDIC BRACE FOR OSTEOPOROSIS**

(30) Priority: 06.04.2023 IT 202300006822
(71) Applicant: F.G.P. S.R.L., 37062 Villafranca Di Verona (Verona) (IT)
(72) Inventor: FERRIGOLO, Moreno, 37062 Villafranca di Verona (VR) (IT); TURRINI, Alberto, 37062 Villafranca di Verona (VR) (IT)
(74) Representative: Sandri, Sandro

(57) **Abstract**

An orthopaedic brace (1) for osteoporosis comprising a longitudinal element (2) positionable, during use, over the patient's spinal column to support the latter; a tensionable lumbar belt (3), coupled with a mid-lower portion (21) of the longitudinal element and configured to wrap around the patient's waist; a pair of tensionable axillary straps (4), each of which is connected to an upper portion (22) and to a substantially central portion (23) of the longitudinal element, each axillary strap comprising an upper band (41) connected to the upper portion and a lower band (42) connected to the central portion; connection means (5) interposed between each upper band and the respective lower band of each axillary strap, wherein the connection means comprise a first element (51) and a second element (52), said elements being reversibly connectable to each other between an engaged configuration and a disengaged configuration.

## Description

### Technical field

The present invention fits into the field of medical devices, in particular orthopaedic devices, for patient rehabilitation and therapy, as well as for an everyday use thereof. In detail, the invention relates to an orthopaedic brace for osteoporosis for the treatment of several pathologies of the neuro-musculoskeletal apparatus of a patient and which is thus capable of assisting the patient during his or her normal motor functions.

In particular, the orthopaedic brace is preferably intended for patients confined to bed and/or patients with reduced motor capacities of the upper limbs.

### Prior art

The use of orthopaedic braces for patient rehabilitation and therapy, such as braces for the treatment of osteoporosis, is presently known.

Typically, an orthopaedic brace for the treatment of osteoporosis is particularly configured to stimulate development of the musculature of the torso, thereby improving posture and consequently reducing the pain that the patient feels every day. Therefore, an orthopaedic brace for osteoporosis is generally configured to be worn as if it were a backpack and, moreover, to follow the anatomy of the back (in particular of the spinal column, with which it is in direct contact).

In detail, therefore, such an orthopaedic brace has a rigid and elastic longitudinal element that can be inserted into a pocket of soft material in order to increase the patient's comfort. The longitudinal element, during use, is configured to be placed over the spinal column so as to provide a support and pushing element. In addition, the brace comprises a plurality of tensionable belts connected with the aforesaid longitudinal element. A belt is generally placed at a lumbar height so as to wrap around the patient's torso. At least one other pair of belts is used as a pair of shoulder straps for putting on the brace and, moreover, for making further adjustments that are particularly effective for the purposes of the treatment of the brace itself.

Generally, the adjustment of the aforesaid shoulder straps (i.e. the tensioning thereof) is carried out by specialised personnel, in particular during a first use of the brace by a patient. At the end of this operation, the length of the upper band and of the lower band is fixed and established, i.e. it cannot be modified except through the intervention of specialised personnel. In detail, the adjustment of the position of the shoulder straps is achieved through the interposition of at least one buckle along each shoulder strap. Document EP2070495 describes an example embodiment of an orthopaedic brace having the aforesaid characteristics.

The main drawback of the known braces described above lies in their use, adjustment, and donning. In greater detail, in order that the brace may effectively perform its function, an ad hoc adjustment of the length of the bands is required, an adjustment operation that is normally long and complex because it requires the manual skill of specialised personnel. Therefore, for the subsequent uses thereof, the brace must be taken off and put on again, taking care not to modify any of the aforesaid adjustments. In addition, the known braces described above and the drawbacks thereof are particularly marked in the event that that they must be worn by patients confined to bed, i.e. patients who are forced to stay in bed and, therefore, in a position and/or with further mobility problems which are impractical even for normal donning operations.

### Objects of the invention

In this context, the technical task at the basis of the present invention is to propose an orthopaedic brace for osteoporosis that makes it possible to obviate and remedy, at least in part, the abovementioned drawbacks of the prior art.

In particular, one object of the present invention is to provide an orthopaedic brace for osteoporosis that is simple to use, in particular that is simple for a patient, in particular also a patient confined to bed, to put on and take off. In addition, the invention also has the object of also making it simpler for a specialised technician to put the orthopaedic brace on a patient and adjust it.

Another object of the present invention is to provide an orthopaedic brace for osteoporosis which is economically advantageous.

A further object of the present invention is to provide an orthopaedic brace for osteoporosis that is structurally simple and, moreover, reliable from a functional viewpoint.

A further object of the present invention is to provide an orthopaedic brace for osteoporosis that is compact and not bulky.

A further object of the present invention is to provide an orthopaedic brace for osteoporosis that has an alternative and/or improved configuration, both in constructive and in functional terms, compared with the known traditional solutions.

### Summary

The stated technical task and specified objects are substantially achieved by an orthopaedic brace for osteoporosis which comprises the technical features disclosed in independent claim number 1. The dependent claims correspond to further advantageous aspects of the invention.

It should be noted that this summary introduces a selection of concepts in simplified form, which will be further elaborated on in the detailed description provided below.

The invention relates to an orthopaedic brace for osteoporosis, in particular for patients confined to bed, comprising a longitudinal element positionable, during use, over the patient's spinal column to support the latter; a tensionable lumbar belt, coupled with a mid-lower portion of the longitudinal element and configured to wrap around the patient's waist; a pair of tensionable axillary straps, each of which is connected to an upper portion and to a substantially central portion of the longitudinal element, each axillary strap comprising an upper band connected to the upper portion and a lower band connected to the central portion; connection means interposed between each upper band and the respective lower band of each axillary strap, wherein the connection means comprise a first element connectable to the upper band and a second element connectable to the lower band, the first element and the second element being reversibly connectable to each other between an engaged configuration and a disengaged configuration so as to make it simpler for a patient to put on the orthopaedic brace.

### Brief description of the drawings

Additional features and advantages of the present invention will emerge more clearly from the approximate and thus non-limiting description of a preferred but not exclusive embodiment of an orthopaedic brace for osteoporosis, as illustrated in the appended drawings, in which:
- figures 1a and 1b illustrate, according to two different perspective views, the orthopaedic brace for osteoporosis of the present invention;

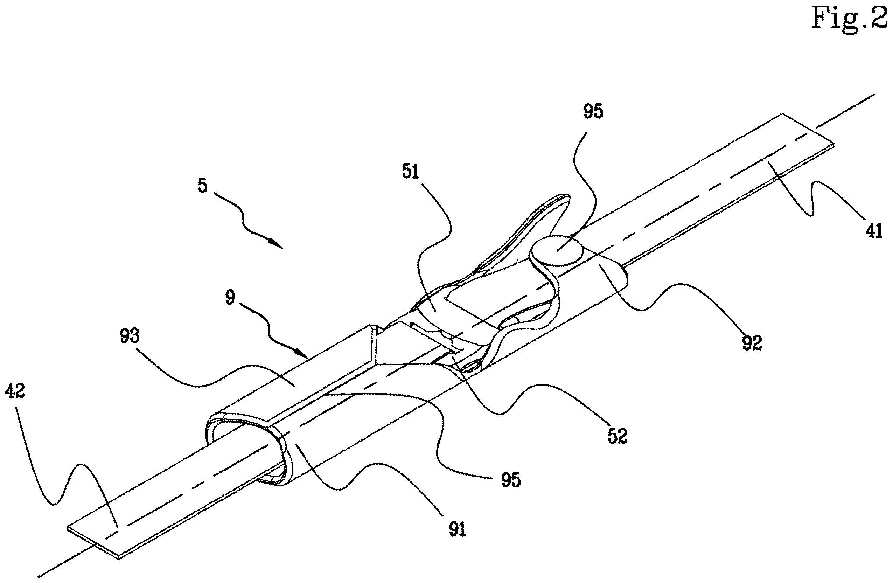

- figure 2 illustrates, according to a perspective view, a component of the orthopaedic brace illustrated in figures 1a and 1b;
- figure 3 illustrates, according to a perspective view, a detail of the component illustrated in figure 2;
- figure 4 illustrates, according to a perspective view, another detail of the component illustrated in figure 2;

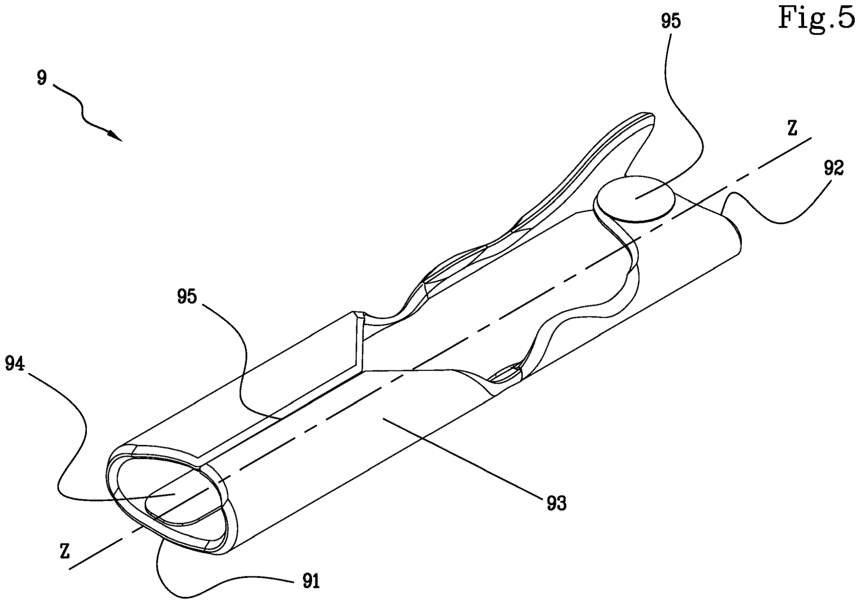

- figure 5 illustrates, according to a perspective view, a further detail of the component illustrated in figure 2;
- figures 6-9 illustrate, according to a respective perspective view, various operating steps in the assembly of the component illustrated in figure 2.

With reference to the drawings, they serve solely to illustrate embodiments of the invention with the aim of better clarifying, in combination with the description, the inventive principles at the basis of the invention.

### Detailed description of at least one embodiment

The present invention relates to an orthopaedic brace for osteoporosis which, with reference to the appended figures, has been generically denoted by the number 1.

Any modifications or variants which, in the light of the description, should be evident to the person skilled in the art must be considered as falling within the scope of protection established by the present invention, according to considerations of technical equivalence.

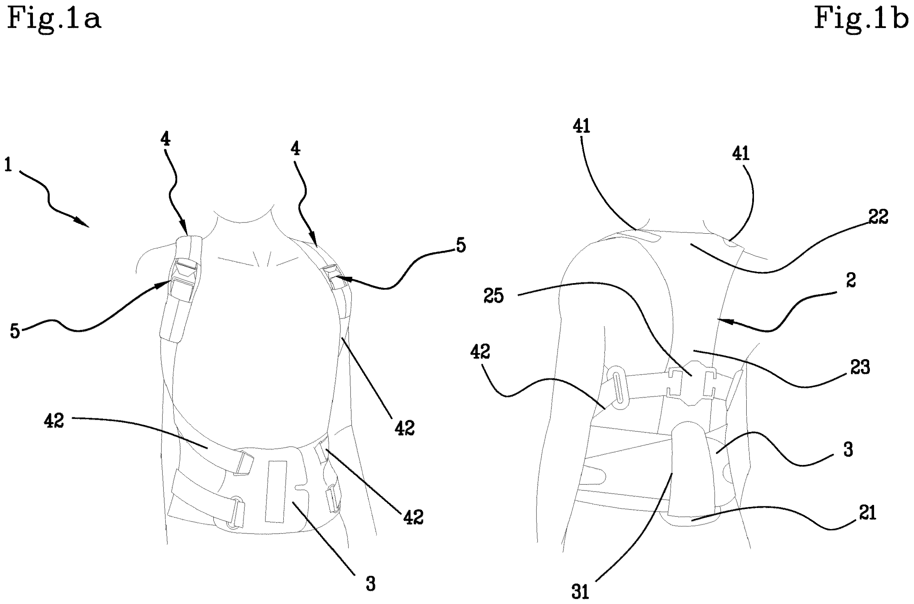

Figures 1a and 1b show an orthopaedic brace 1 for osteoporosis, in particular for patients confined to bed, comprising a longitudinal element 2 positionable, during use, over the patient's spinal column to support the latter.

Preferably, the longitudinal element 2 is made with a flexible material in order to be adapted to the shape of a patient's spinal column. At the same time, the longitudinal element 2 is made with a resistant material, in particular one resistant to traction, in order to impart to the longitudinal element 2 itself mechanical properties of resistance such as to support the aforesaid spinal column.

Preferably, the longitudinal element 2 extends between an upper portion 22, arranged during use in proximity to the patient's neck; and a mid-lower portion 21 arranged during use in proximity to the patient's waist.

The longitudinal element 2 further comprises a central portion 23 arranged between the upper portion 22 and the mid-lower portion 21. Preferably, the central portion 23 is slidably associated with a plate 25, which is movable along the longitudinal element 2 so that the position of the plate 25 can be varied according to the treatment the patient must undergo and, therefore, also based on the length of the patient's torso.

The orthopaedic brace 1 further comprises a tensionable lumbar belt 3, coupled with the mid-lower portion 21 of the longitudinal element 2 and configured to wrap around the patient's waist.

In greater detail, the lumbar belt 3 is preferably slidably associated with the longitudinal element 2, for example by passing the lumbar belt 3 through a pocket 31 (not illustrated in the appended figures) obtained in the mid-lower portion 21. In this manner, the lumbar belt 3 is adaptable according to the needs of the user patient.

Preferably, the lumbar belt 3 has a substantially constant width.

The orthopaedic brace 1 further comprises a pair of tensionable axillary straps 4, each of which is connected to the upper portion 22 and to the substantially central portion 23 of the longitudinal element 2.

Preferably, the axillary straps 4 are substantially identical, i.e. they preferably have the same length and the same width. Preferably, the width of the axillary straps 4 is smaller than the width of the lumbar belt 3.

In greater detail, each axillary strap 4 comprises an upper band 41 connected to the upper portion 22 and a lower band 42 connected to the central portion 23.

The orthopaedic brace 1 further comprises connection means 5 interposed between each upper band 41 and the respective lower band 42 of each axillary strap 4. In particular, the connection means 5 comprise a first element 51 connectable to the upper band 41 and a second element 52 connectable to the lower band 42.

In accordance with the embodiment illustrated in the appended figures, the first element 51 preferably has a substantially planar, e.g. plate-like, prevalent shape, and is preferably made of plastic or metal material, such as steel, in order to impart resistance and rigidity to the first element 51 itself.

Similarly, the second element 52 preferably has a substantially planar, e.g. plate-like, prevalent shape, and is preferably made of plastic or metal material, such as steel, in order to impart resistance and rigidity to the second element 52 itself.

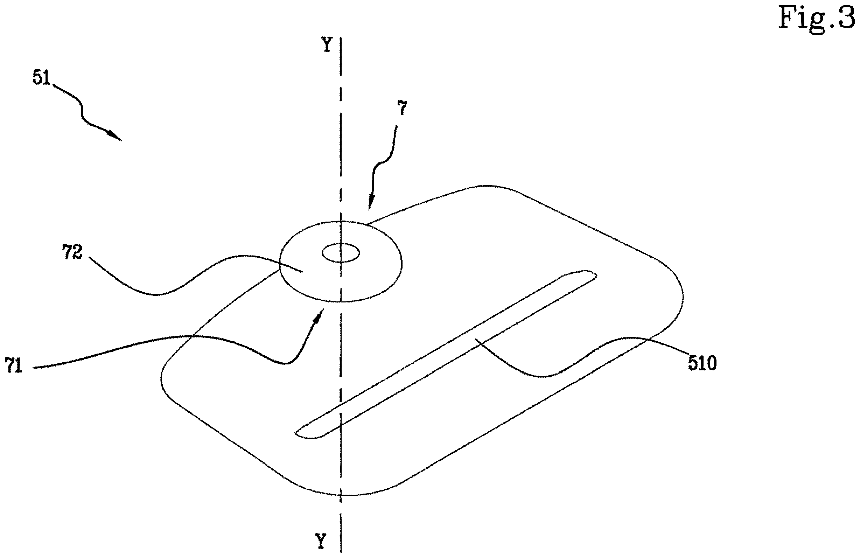

Preferably, the first element 51 defines a first opening 510, preferably a through opening (see FIG.3), for example a slot, a slit or the like, shaped so as to be mechanically connected to the upper band 41.

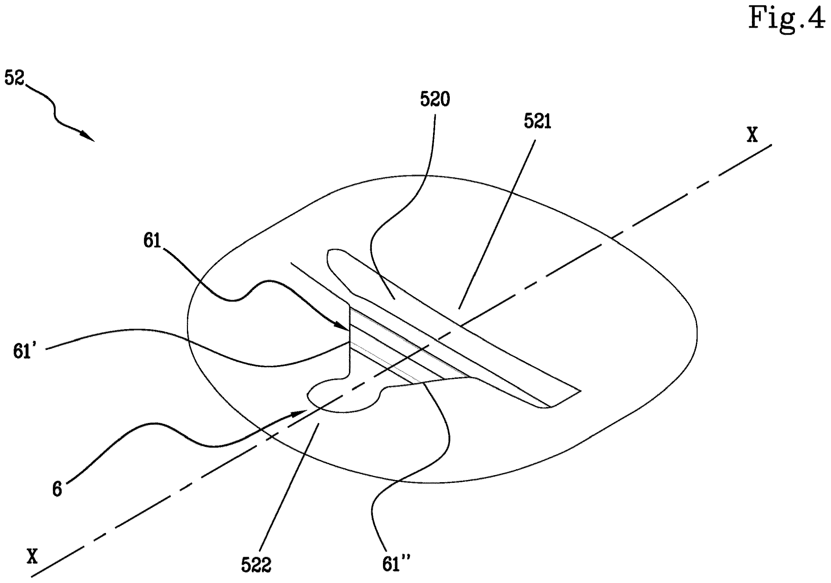

Preferably, the second element 52 defines a second opening 520, preferably a through opening (see FIG.4), extending along an axis X between a first end 521 and a second end 521.

In greater detail, at the first end 521, the second opening 520 is shaped so as to be mechanically connected to the lower band 42.

At the second end 522, by contrast, the second opening 520 is shaped so as to be coupled with the first element 51 (as described in greater detail below).

In greater detail, the first element 51 and the second element 52 are reversibly connectable to each other between an engaged configuration and a disengaged configuration so as to make it simpler for a technician to put the orthopaedic brace 1 on a patient.

Advantageously, from an operational viewpoint, the patient and/or specialised personnel (for example a doctor, a nurse, a physiotherapist or the like) engages and/or disengages, according to need, the upper band 41 to or from the lower band 42 of each axillary strap 4, without having to make any type of change to the length of the bands in relation to the initial length adjustment.

In particular, this operation of engaging/disengaging and thus of putting the brace on a patient confined to bed is particularly easy.

Even more advantageously, in fact, the latter can use the brace in complete autonomy even in a lying position, the result being a rapid and simple use of the orthopaedic brace 1 itself.

In accordance with a preferred aspect of the invention, in the engaged configuration the first element 51 and the second element 52 of the connection means 5 are shaped so as to define a mating coupling. In accordance with a further aspect of the invention, in the engaged configuration the first element 51 and the second element 52 of the connection means 5 are shaped so as to define an interlock coupling.

Advantageously, in the engaged configuration the first element 51 and the second element 52 are stably and securely coupled, thus avoiding the possibility of an unwanted decoupling of the aforesaid elements during the use of the brace 1.

In accordance with a preferred embodiment of the connection means 5, the second element 52 preferably has at least one seat 6 and the first element 51 preferably comprises at least one projecting portion 7 configured to couple with said seat 6.

Preferably, the seat 6 is obtained at the second end 522 of the second opening 520 of the second element 52.

Preferably, the seat 6 preferably has a lead-in zone 61 configured to assist the insertion of the projecting portion 7 of the first element 51.

In greater detail, the lead-in zone 61 preferably comprises at least two inclined sections 61', 61"; preferably arranged symmetrically relative to the axis X and configured to facilitate the sliding of the projecting portion 7 into the seat 6.

Advantageously, the coupling between the first element 51 and the second element 52 is simple and safe, since the projecting portion 7 is exclusively directed towards/conveyed into the seat 6.

Preferably, the projecting portion 7 comprises a base portion 71 configured to couple with said seat 6 and a head portion 72 mechanically connected to the base portion.

Preferably, the base portion and the head portion 72 of the projecting portion 7 extend along an axis Y which is substantially orthogonal to the surface of the first element 51 (see FIG.3).

In greater detail, the head portion 72 has a larger transverse dimension than the base portion in order to retain the projecting portion 7 in position in the seat 6.

Advantageously, the coupling between the first element 51 and the second element 52 is wholly reliable, i.e. there is no possibility that the base portion 71 of the projecting portion 7 will come out of the seat 6.

In addition, an axillary strap 4 created through the reversible mechanical coupling between due bands, an upper one 41 and a lower one 42 associated with the same shoulder of the brace, allows for providing a double adjustment of the tensioning of the aforesaid bands 41, 42 and, therefore, of the entire axillary strap 4 irrespective of the condition of engagement or disengagement of the aforesaid bands to each other.

Even more advantageously, the coupling and decoupling between the upper 41 and lower 42 bands can also be easily achieved by the patient who must, respectively, put on or take off the orthopaedic brace 1.

In particular, the mechanical engagement/disengagement system created by means of the seat 6 of the second element 52 and the projecting portion 7 of the first element 51 can also be easily implemented by a patient having reduced mobility since, in the case both of the engagement and disengagement operation, it is sufficient to keep one band, for example the lower one 42, firmly in position whilst the other band, in this case the upper one 41, is maintained overlapping. The projecting portion 7 can thus be easily constrained to or released from the seat 6 without losing control of the bands 41, 42 and without altering the adjustments made previously. Advantageously, in fact, the first and second elements 51, 52 are maintained in an engaged configuration thanks to the tensioning induced by the bands 41, 42 themselves on, respectively, the projecting portion 7 and the seat 6 constrained to each other due to mechanical interference.

In other words, in accordance with the invention, a user will be able to engage and disengage the upper and lower bands 41, 42 thanks to a single simple movement.

In contrast, the known systems of engagement and disengagement of the bands are built with more complicated mechanisms that can reduce the integrity of the bands, for example systems with a buckle and pin (similar to a belt), and/or which can require a major effort - i.e. the application of a large pressing force - on the part of a patient with reduced mobility in order to achieve the passage from the engaged configuration to the disengaged one and, moreover, to achieve the separation of the bands.

Preferably, the seat 6 and the base portion 72 have a substantially similar shape, in particular substantially circular. Preferably, the extent of the seat 6 is greater than the extent of the base portion 72 in order to allow the aforesaid coupling between the first element 51 and the second element 52.

In accordance with one embodiment of the connection means 5, not illustrated in the appended figures, the second element 52 preferably defines a housing volume, and the first element 51 is configured to be snap fitted into the housing volume of the second element 52.

In greater detail, the second element 52 comprises a third opening, preferably fashioned at the surface of the second element 52 itself and configured to receive a retaining means (described in greater detail below).

In greater detail, the first element 51 preferably comprises a retaining means, for example at least one retaining tab, preferably extending along a direction that is substantially orthogonal to the direction of housing of the first element 51 with the second element 52, and preferably configured to be housed in the third opening, in order to retain the first element 51 in position when it is housed in the housing volume of the second element 52.

In other words, the aforesaid unillustrated embodiment of the connection means 5 is preferably of the snap-fit type.

In accordance with a further embodiment of the connection means 5, not illustrated in the appended figures, the first element 51 preferably comprises a pin rotatably connected to the upper band 41 and configured to be coupled mechanically with an opening obtained at the second element 52 of the lower band 42 so as to retain the bands in a stable reciprocal position.

Preferably, the pin has a substantially cylindrical and preferably substantially constant shape along the whole of its length.

Preferably, the pin is made of metal material, such as, in particular, steel.

In accordance with the embodiment illustrated in the appended figures, the orthopaedic brace 1 preferably comprises at least one covering element 9 configured to at least partially wrap around said connection means 5 so as to be interposed between said connection means 5 and the patient's shoulder.

During use, the covering element 9 is preferably wrapped at least partially around the connection means 5, preferably slidably.

During use, the covering element 9 is preferably wrapped at least partially around the upper band 41 and the lower band 42, preferably slidably.

Preferably, the covering element 9 extends along an axis Z, preferably substantially parallel to the axis X, between a first end 91 and a second end 92, and preferably comprises at least two components, in particular:
- a base 93, preferably made of textile material, in particular padded, and preferably formed in such a way as to define, during use, a preferably substantially tubular shape;
- at least one insert 94, mechanically associated with the base 93, which preferably extends between the first end 91 and the second end 92 and is configured to impart rigidity and robustness to the covering element 9.

Advantageously, the at least one insert 94 imparts rigidity and robustness to the base 93 and facilitates the sliding of the bands (i.e. of the upper band 41 and lower band 42) through the aforesaid covering element 9.

Preferably, the base 93 comprises at least one gripping zone 95, preferably two gripping zones 95, preferably located at the first end 91 and second end 92 of the covering element 9.

In greater detail, the gripping zones 95 preferably define a gripping system of a mechanical type, such as, in particular, a button and/or Velcro^{®}. Advantageously, the connection/disconnection of the covering element 9 is reversible, resulting in a rapid and simple wrapping of the covering element 9 around the bands of the brace 1. In this manner, the covering element 9 can be easily positioned as desired by the patient.

The at least one insert 94 is preferably made of polymeric material and is mechanically connected to the base 93, preferably by stitching or welding, in particular highfrequency (HF) welding.

As regards an example of assembly of the orthopaedic brace 1, or at least of the axillary straps 4 and the connection means 5, it derives directly from what was described above and is referred to below.

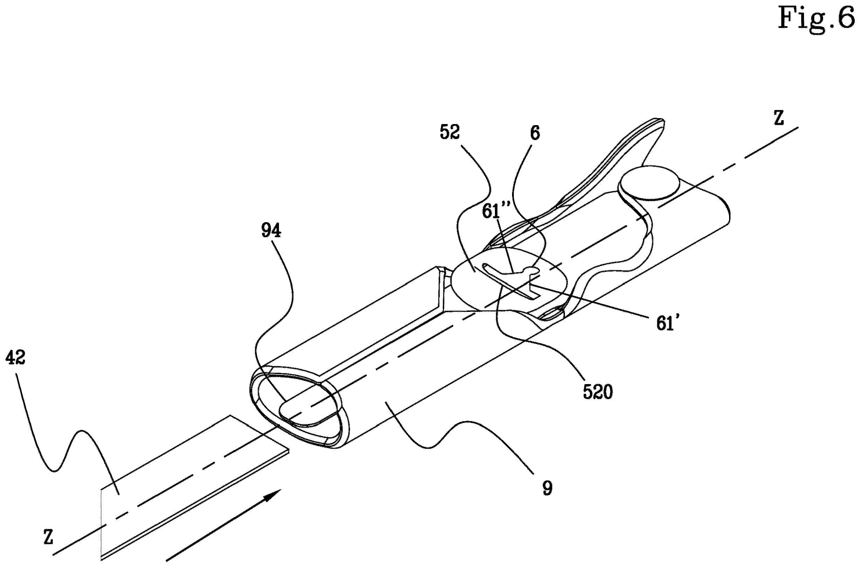

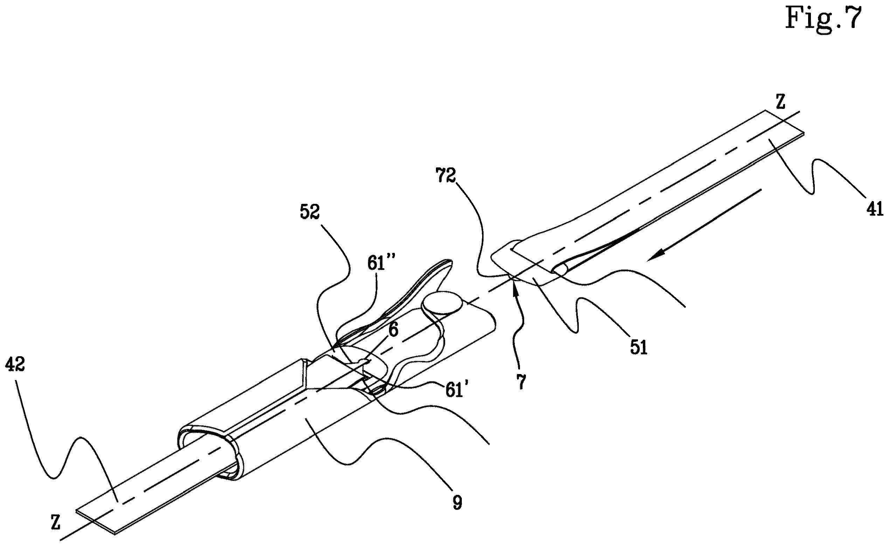

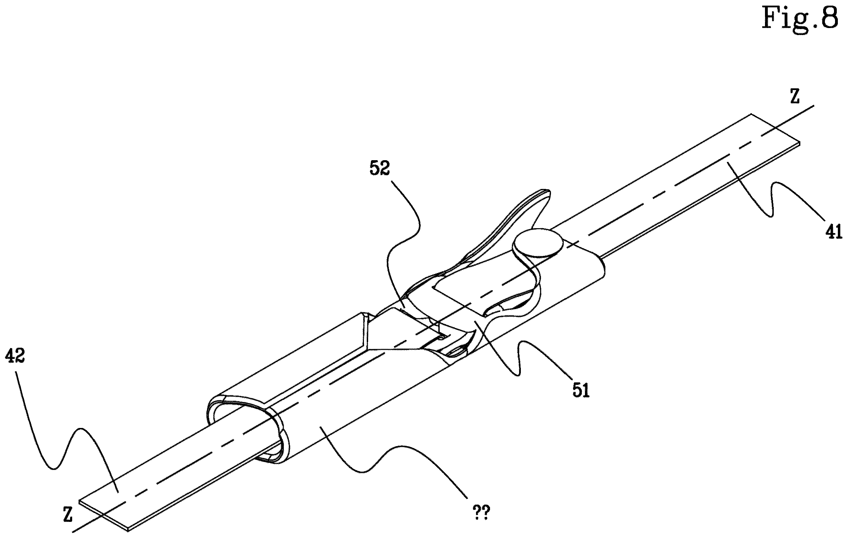

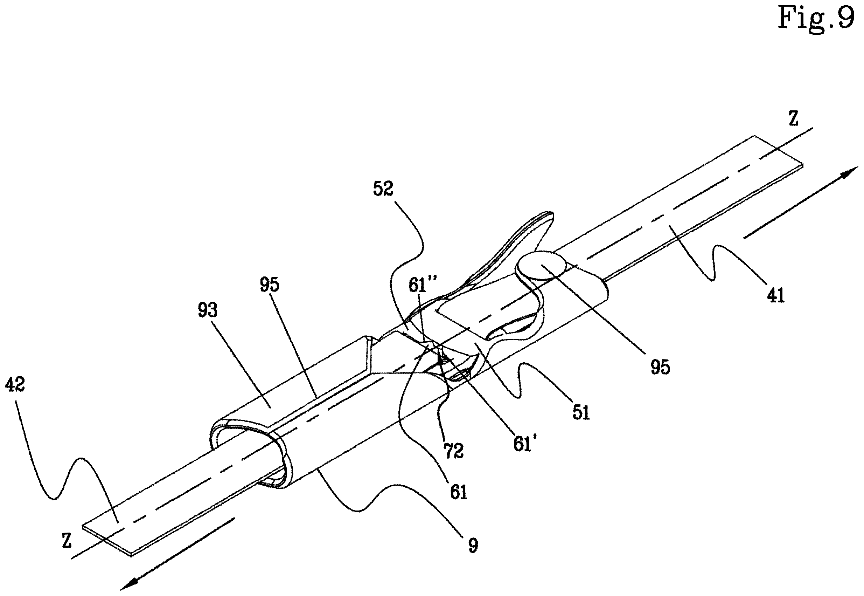

Figures 6-9 show the salient operating steps of assembling the straps 4 of an orthopaedic brace 1 in accordance with the invention.

First, it is necessary to prepare the aforesaid connection means 5 and, moreover, at least the upper band 41 and the lower band 42 for each axillary strap 4.

At this point, the lower band 42 is constrained to the second element 52 of the connection means 5, whilst the upper band 41 is constrained to the first element 51 of the connection means 5.

Preferably, the bands are passed through specific openings 510, 520 (preferably shaped like grooves) obtained on the respective first and second elements 51, 52 of the connection means 5. Optionally, the bands can be folded over themselves around the aforesaid slot/groove in order to remain solidly in position relative to the first or second element 51, 52.

If the orthopaedic brace 1 also comprises the covering element 9, at least the lower band 42 is passed beforehand through the tubular opening of the covering element 9 before a connection is made with the respective second element 52.

Finally, once the upper and lower bands 41, 42 are connected to the respective first and second elements 51, 52, it is sufficient complete the mating coupling between the aforesaid first and second elements 51, 52 in order to join the bands together and obtain the complete strap 4.

## Claims

1. An orthopaedic brace (1) for osteoporosis, in particular for patients confined to bed, comprising:
- a longitudinal element (2) positionable, during use, over the patient's spinal column to support the latter;
- a tensionable lumbar belt (3), coupled with a mid-lower portion (21) of said longitudinal element (2) and configured to wrap around the patient's waist;
- a pair of tensionable axillary straps (4), each of which is connected to an upper portion (22) and to a substantially central portion (23) of said longitudinal element (2), each axillary strap (4) comprising an upper band (41) connected to said upper portion (22) and a lower band (42) connected to said central portion (23);
- connection means (5) interposed between each upper band (41) and the respective lower band (42) of each axillary strap (4), wherein said connection means (5) comprise a first element (51) connectable to said upper band (41) and a second element (52) connectable to said lower band (42), said first element (51) and said second element (52) being reversibly connectable to each other between an engaged configuration and a disengaged configuration so as to make it simpler to put said orthopaedic brace (1) on a patient.
wherein said second element (52) has at least one seat (6) and said first element (51) comprises at least one projecting portion (7) configured to couple with said seat (6), said projecting portion (7) comprising:
- a base portion configured to couple with said seat (6);
- a head portion (72) mechanically connected to said base portion;
wherein said head portion (72) has a larger transverse dimension than said base portion in order to retain said projecting portion in position in said seat (6).

2. The orthopaedic brace (1) according to claim 1, **characterised in that** said first element (51) and said second element (52) of said connection means (5) in said engaged configuration are shaped so as to define a mating coupling.

3. The orthopaedic brace (1) according to claim 1 or 2, **characterised in that** said first element (51) and said second element (52) of said connection means (5) in said engaged configuration are shaped so as to define an interlock coupling.

4. The orthopaedic brace (1) according to one or more of the preceding claims, **characterised in that** said seat (6) has a lead-in zone (61) configured to assist the insertion of said projecting portion (7) of said first element (51).

5. The orthopaedic brace (1) according to one or more of the preceding claims, **characterised in that** said lead-in zone (61) comprises at least two inclined sections (61', 61") arranged symmetrically relative to an axis X and configured to facilitate the sliding of said projecting portion (7) into said seat (6).

6. The orthopaedic brace (1) according to claims 1 to 3, **characterised in that** said second element (52) defines a housing volume, said first element (51) being configured to be snap fitted into said housing volume of said second element (52).

7. The orthopaedic brace (1) according to claims 1 to 3, **characterised in that** said first element (51) comprises a pin rotatably connected to said upper band (41) and configured to be coupled mechanically with an opening obtained at said second element (52) of said lower band (42) so as to retain said bands in a stable reciprocal position.

8. The orthopaedic brace (1) according to one or more of the preceding claims, **characterised in that** it comprises at least one covering element (9) configured to at least partially wrap around said connection means (5) so as to be interposed between said connection means (5) and the shoulders of said patient.
